# EUROPÄISCHE PATENTSCHRIFT

(11) **EP 3 538 014 B1**
(45) Veröffentlichungstag und Bekanntmachung des Hinweises auf die Patenterteilung: **28.06.2023**
(21) Anmeldenummer: 17829123.3
(22) Anmeldetag: 08.11.2017
(51) Int. Cl.: A61B 46/00

(54) **SCHUTZÜBERZUG FÜR EIN TEIL EINES MEDIZINGERÄTS**
PROTECTIVE COVER FOR A PART OF A MEDICAL DEVICE
HOUSSE PROTECTRICE POUR UNE PARTIE D'UN APPAREIL MEDICAL

(30) Priorität: 11.11.2016 DE 102016013394
(43) Veröffentlichungstag der Anmeldung: 18.09.2019
(73) Patentinhaber: Drägerwerk AG & Co. KGaA, 23558 Lübeck (DE)
(72) Erfinder: BARTEN, Ronny, 23562 Lübeck (DE); HILLEKE, Christian, 23558 Lübeck (DE); WOTHA, Gerd, 23626 Warnsdorf (DE)
(86) Internationale Anmeldenummer: PCT/EP2017/001296
(87) Internationale Veröffentlichungsnummer: WO 2018/086736

(56) Entgegenhaltungen:
- EP-A1- 3 056 164
- WO-A1-89/07900
- WO-A1-2015/158397
- DE-A1-102008 061 362
- US-A- 5 355 292
- US-A1- 2003 014 834
- US-A1- 2014 226 304

## Beschreibung

Die Erfindung betrifft eine als Schutzüberzug fungierende Vorrichtung (Schutzvorrichtung) für ein Teil eines Medizingeräts, insbesondere für einen Handgriff eines Medizingeräts, zum Beispiel eine zur Verwendung in einem Operationssaal bestimmte Leuchte (OP-Leuchte).

Medizingeräte und damit auch zur Verwendung in einem Operationssaal bestimmte Medizingeräte müssen vom medizinischen Personal oftmals während der Verwendung, zum Beispiel während einer Operation, manuell verstellt oder justiert werden. Dafür besitzen solche Medizingeräte Handgriffe oder dergleichen, die steril sein müssen, um Übertragungsinfektionen durch einen Anwender auf einen Patienten oder umgekehrt zu verhindern. Üblicherweise werden zu diesem Zweck solche Handgriffe zum Beispiel vor jeder Operation gewechselt oder es wird eine Schutzvorrichtung in Form einer sterilen Abdeckung für den Einmalgebrauch oder in Form eines Einmalüberzugs auf dem Handgriff angebracht.

Solche Einmalüberzüge bestehen zumeist aus einem Beutel aus einem Kunststoff, welcher über den jeweiligen Handgriff geschoben wird. Am offenen Ende wird der Beutel durch einen stabilen Anteil offen gehalten. Mittels des stabilen Anteils wird die Schutzvorrichtung oftmals kraft- oder formschlüssig mit dem Handgriff verbunden. Zusätzlich oder alternativ wird auch ein beim Aufsetzen einer Schutzvorrichtung auf den jeweiligen Handgriff resultierendes Vakuum zur Halterung an dem Handgriff genutzt, indem durch gute Abdichtung ein Luftzufluss in das Innere der Schutzvorrichtung verhindert wird.

Bekannte Schutzvorrichtungen sind je nach Ausführung teuer in der Herstellung, insbesondere weil bereits die Einzelbauteile, speziell Einzelbauteile in Form von zum Beispiel tiefgezogenen Kunststoffteilen, teuer sind und mehrteilige Schutzvorrichtungen mittels aufwändiger Verfahren miteinander verbunden werden müssen.

Gerade bei kraftschlüssigen Verbindungen zum jeweiligen Handgriff ist die Anbringung bekannter Schutzvorrichtungen an einem Medizingerät mitunter stark erschwert, da eine Anbringung mit nur einer Hand sehr aufwändig ist und teilweise muss daher eine weitere Person an nicht sterilen Bauteilen des Medizingerätes gegenhalten.

Des Weiteren ist nach einer Benutzung bekannter Schutzvorrichtungen nicht ohne weiteres erkennbar, ob das jeweilige Produkt bereits montiert war. Bei Einmalprodukten besteht somit die latente Gefahr, durch fahrlässige oder mutwillige Mehrfachverwendung Patienten und Anwender zu infizieren.

Schließlich haben bekannte Schutzvorrichtungen in einer jeweiligen Verpackung ein vergleichsweise großes Volumen. Dadurch steigen die Sterilisationskosten, die Kosten für die Sterilverpackung und der Platzbedarf für die Lagerung, wobei Letzteres aufgrund des ungleich stärker beschränkten Platzangebots besonders kritisch bei einer Lagerung im OP (Operationssaal) oder in einer Operationsabteilung ist.

D1 (US 5355292) zeigt eine Befestigungsvorrichtung (lighting fixture 10) für eine Beleuchtungseinrichtung (surgical lighting), welche ein Chirurg 16 verwenden kann. An einem Körper 12 ist eine Anordnung 14 mit einem Handgriff 18 und einem sterilen wegwerfbaren Überzug 20 angeordnet. Der Handgriff 18 hat einen Griffbereich (handle grip portion 26) und einen Basisbereich (central stud 24, adapter 22) mit einer umlaufenden starren Scheibe 36, einer weiteren starren Scheibe 38 und einer umlaufenden Aussparung 42. Der Überzug 20 umfasst einen elastischen Überzug 52 über den Griffbereich 26, einen starren umlaufenden Flansch (cover flange 58) und vier hakenförmige Vorsprünge 62, die in die Aussparung 42 eingreifen können.

EP 3056164 A1 zeigt eine Schutzvorrichtung für eine Leuchte 10, welche einen Operationssaal beleuchten kann. Um Parameter der Leuchte 10 zu verändern, kann ein Benutzer einen Hebel (knob 42) drehen. Durch diese Drehung wird ein Basisteil 40 mit einer Plattform 52 gedreht. In das Basisteil 40 sind Betätigungselemente 114 eingelassen. Ein Überzug (cover 44) besitzt einen Teil 100 für den Hebel 42 und einen weiteren Teil 102 für die Plattform 52. Um den Überzug 44 zu befestigen, greift ein federbelasteter Hebel 406 in eine korrespondierende Aussparung 404 ein.

Zusammenfassung Anspruch 1 definiert die Erfindung und die abhängigen Ansprüche offenbaren Ausführungsformen. Eine Aufgabe der vorliegenden Erfindung besteht ausgehend von den oben skizzierten Beobachtungen bezüglich bekannter Schutzvorrichtungen darin, eine Schutzvorrichtung mit den Merkmalen des Oberbegriffs des Anspruchs 1 für einen Handgriff eines Medizingeräts anzugeben, die sich bezüglich einzelner oder mehrerer der genannten Nachteile durch Verbesserungen auszeichnet.

Erfindungsgemäß wird diese Aufgabe mittels einer Schutzvorrichtung für einen Handgriff eines Medizingeräts mit den Merkmalen des Anspruchs 1 gelöst. Dazu ist bei einer Schutzvorrichtung für einen Handgriff eines Medizingeräts vorgesehen, dass die Schutzvorrichtung ein erstes, flexibles Teil und ein zweites, festes Teil umfasst, dass das flexible Teil bei einer am Handgriff angebrachten Schutzvorrichtung zumindest einen Griffbereich des Handgriffs vollständig abdeckt, dass das feste Teil lösbar mit einem Basisbereich des Handgriffs in Eingriff ist und dass das feste Teil zumindest ein Verriegelungselement zur lösbaren Fixierung am Basisbereich umfasst.

Die hier vorgeschlagene Schutzvorrichtung für einen Handgriff eines Medizingeräts umfasst demnach ein festes, zur Fixierung der Schutzvorrichtung an dem Handgriff bestimmtes Teil sowie ein flexibles, zum Abdecken zumindest des Griffbereichs des Handgriffs bestimmtes, beutel- oder tütenartiges Teil, wobei beide Teile miteinander verbunden sind und wobei das feste Teil wenigstens ein Verriegelungselement aufweist, das mit einem Gegenstück des Handgriffs lösbar verbindbar, zum Beispiel rastend lösbar verbindbar ist. Typische Medizingeräte, an deren Handgriffen eine solche Schutzvorrichtung vorteilhaft verwendbar ist, sind zum Beispiel Operationsleuchten (OP-Leuchten) und Monitoraufnahmen.

Ein Vorteil der Neuerung besteht darin, dass die Schutzvorrichtung leicht, insbesondere mit nur einer Hand, am jeweiligen Handgriff anbringbar und an diesem fixierbar ist, ohne dass dabei die Notwendigkeit besteht, die Umgebung des Handgriffs und das Medizingerät selbst und damit nicht sterile Teile berühren zu müssen. Darüber hinaus ergibt sich durch die Aufteilung in einen flexiblen Teil und einen festen Teil eine vorteilhaft kleine Packungsgröße, wobei im Wesentlichen der feste Teil das benötigte Volumen bestimmt.

Lösungsgemäß weist die Schutzvorrichtung in ihrem festen Teil ein zum Zusammenwirken mit einem Gewinde oder einem Profil im Basisbereich des jeweiligen Handgriffs bestimmtes Gewinde oder Profil auf. Das Profil am festen Teil und das Profil am Basisteil sind in der Form oder nach Art eines Bajonettverschlusses ausgestaltet. Mittels eines solchen Gewindes oder Profils lässt sich eine besonders sichere Fixierung der Schutzvorrichtung am jeweiligen Handgriff gewährleisten.

Vorteilhafte Ausgestaltungen der Erfindung sind Gegenstand der Unteransprüche. Dabei verwendete Rückbeziehungen weisen auf die weitere Ausbildung des Gegenstandes des Hauptanspruches durch die Merkmale des jeweiligen Unteranspruches hin. Sie sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche sowie der Beschreibung bei einer näheren Konkretisierung eines Merkmals in einem nachgeordneten Anspruch davon auszugehen, dass eine derartige Beschränkung in den jeweils vorangehenden Ansprüchen sowie einer allgemeineren Ausführungsform der gegenständlichen Schutzvorrichtung nicht vorhanden ist. Jede Bezugnahme in der Beschreibung auf Aspekte nachgeordneter Ansprüche ist demnach auch ohne speziellen Hinweis ausdrücklich als Beschreibung optionaler Merkmale zu lesen.

Bei einer Ausführungsform der Schutzvorrichtung weist diese Drehhilfen an ihrem festen Teil auf. Solche Drehhilfen erleichtern die zum Fixieren der Schutzvorrichtung am jeweiligen Handgriff notwendige Drehbewegung des festen Teils relativ zum Basisbereich des Handgriffs.

Die Fixierung der Schutzvorrichtung an dem jeweiligen Handgriff lässt sich besonders leicht und unkompliziert aufheben, wenn die Schutzvorrichtung am festen Teil eine Entriegelungsvorrichtung zum Lösen des Verriegelungselements aus einer Fixierung im Basisbereich des Handgriffs aufweist.

Bei einer besonderen Ausführungsform der Schutzvorrichtung weist diese am festen Teil zumindest eine der Entriegelungsvorrichtung räumlich zugeordnete Sollbruchstelle auf. Bei einer Betätigung der Entriegelungsvorrichtung, zum Beispiel einem mit der Hand des Anwenders ausgeübten Zug an der Entriegelungsvorrichtung, gibt die oder zumindest eine Sollbruchstelle nach, so dass eine irreversible Verformung des festen Teils der Schutzvorrichtung resultiert. Anhand einer solchen Verformung ist jederzeit sicher erkennbar, ob die Schutzvorrichtung bereits in Gebrauch war. Ein nicht vorgesehener Mehrfachgebrauch der Schutzvorrichtung kann damit wirksam verhindert werden.

Eine besondere Ausführungsform der Schutzvorrichtung zeichnet sich durch ein starres, tellerförmiges festes Teil aus. Mit der Tellerform ist das feste Teil besonders gut an die übliche Form eines Basisbereichs eines Handgriffs an einem Medizingerät angepasst. Darüber hinaus lässt sich das tellerförmige feste Teil besonders gut am Basisbereich und damit die Schutzvorrichtung insgesamt besonders gut am Handgriff anbringen. Ein solches festes Teil wird im Weiteren demgemäß auch als Montageteller bezeichnet. Die Tellerform - also die runde Form - des festen Teils gewährleistet zudem eine allseitig gute Führung des flexiblen Teils durch das feste Teil, so dass das flexible Teil bei einer an einem Handgriff angebrachten Schutzvorrichtung allseitig gut am Griffbereich des Handgriffs anliegt.

Bei einer alternativen Ausführungsform der Schutzvorrichtung umfasst diese eine zumindest in Teilbereichen mit dem flexiblen Teil verbundene Klammer als festes Teil. Eine solche Klammer ist im Rahmen ihrer Materialeigenschaften elastisch beweglich und ermöglicht beim Zusammendrücken zum Beispiel ein Öffnen des mit der Klammer verbundenen flexiblen Teils. Dies erleichtert die Anbringung der Schutzvorrichtung an dem jeweiligen Handgriff, nämlich das Überziehen des flexiblen Teils über den Griffbereich des Handgriffs. Zudem ist die Klammer ein flaches Bauteil und entsprechend ergibt sich für die Schutzvorrichtung im nicht benutzten Zustand eine vorteilhaft kleine Packungsgröße, wobei im Wesentlichen die Klammer die benötigte Grundfläche der Verpackung bestimmt.

Bei einer Ausführungsform einer Schutzvorrichtung mit einer Klammer als festem Teil weist diese zur lösbaren Fixierung der Schutzvorrichtung an einem jeweiligen Handgriff an der Klammer zumindest einen als Verriegelungselement fungierenden Verriegelungshaken auf, der beim Fixieren an dem Handgriff in einen Verriegelungsbereich des Handgriffs eingreift.

Bei einer weiteren besonderen Ausführungsform einer Schutzvorrichtung mit einer Klammer als festem Teil weist diese zwei an der Klammer angelenkte und mit dem flexiblen Teil in dessen Randbereich zumindest abschnittsweise verbundene Bügel auf. Diese Bügel öffnen den flexiblen Teil beim Zusammendrücken der Klammer in einer definierten Art und Weise und halten zudem das flexible Teil allseitig an dessen Rändern, so dass das flexible Teil bei einer an einem Handgriff angebrachten Schutzvorrichtung allseitig gut zumindest an dessen Griffbereich anliegt.

Insgesamt ist die hier vorgeschlagene Neuerung auch ein System, welches einerseits eine Schutzvorrichtung der hier und im Folgenden beschriebenen Art und andererseits einen Handgriff eines Medizingeräts umfasst, wobei der Handgriff einen Griffbereich und einen Basisbereich aufweist. Bei einer besonderen Ausführungsform eines solchen Systems ist der Basisbereich des Handgriffs zur lösbaren Fixierung des festen Teils der Schutzvorrichtung am Handgriff eingerichtet und weist dafür zumindest einen Verriegelungsbereich zur Aufnahme des Verriegelungselements der Schutzvorrichtung auf. Bevorzugt ist wenigstens eine Sollbruchstelle vorgesehen, und derart ausgeführt, dass diese bei einem Entriegeln der Schutzvorrichtung bricht, so den bereits erfolgten Gebrauch der Schutzvorrichtung deutlich anzeigt und eine erneute Verwendung der Vorrichtung ausschließt.

Bei einer weiteren Ausführungsform eines solchen Systems weist der Verriegelungsbereich einen Fügebereich, einen Rastbereich und dazwischen einen Anlaufbereich mit einer Anlaufschräge auf. Der Fügebereich ist beim Verbinden der Schutzvorrichtung mit dem Handgriff zur Aufnahme des Verriegelungselements der Schutzvorrichtung bestimmt und nimmt beim anfänglichen Verbinden der Schutzvorrichtung mit dem Handgriff das Verriegelungselement auf. Mittels der Anlaufschräge ist das Verriegelungselement der Schutzvorrichtung beim Verbinden der Schutzvorrichtung mit dem Handgriff und beim Drehen des festen Teils relativ zum Basisbereich des Handgriffs auslenkbar und wird beim Verbinden der Schutzvorrichtung mit dem Handgriff und beim Drehen des festen Teils relativ zum Basisbereich des Handgriffs ausgelenkt. Beim Weiterdrehen des festen Teils gelangt das Verriegelungselement von der Anlaufschräge in den Rastbereich und ist dort zur rastenden Fixierung des festen Teils am Basisbereich des Handgriffs wirksam.

Nachfolgend werden Ausführungsbeispiele der Erfindung anhand von Zeichnungen näher erläutert. Einander entsprechende Gegenstände oder Elemente sind in allen Figuren mit den gleichen Bezugszeichen versehen.

Das oder jedes Ausführungsbeispiel ist nicht als Einschränkung der Erfindung zu verstehen. Vielmehr sind im Rahmen der vorliegenden Offenbarung Abänderungen und Modifikationen möglich, insbesondere solche Varianten und Kombinationen, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen für den Fachmann im Hinblick auf die Lösung der Aufgabe entnehmbar sind und durch kombinierbare Merkmale zu einem neuen Gegenstand führen.

Es zeigen:
- Figur 1: eine Schutzvorrichtung für einen Handgriff eines Medizingeräts, gemäß der Erfindung,
- Figur 2: einen für eine Anbringung einer Schutzvorrichtung gemäß Figur 1 bestimmten Handgriff,
- Figur 3: einen Montageteller der Schutzvorrichtung gemäß Figur 1,
- Figur 4: eine an einem Handgriff angebrachte Schutzvorrichtung gemäß Figur 1,
- Figur 5: eine alternative Ausführungsform eines Montagetellers für eine Schutzvorrichtung gemäß Figur 1,
- Figur 6: eine weitere Schutzvorrichtung für einen Handgriff eines Medizingeräts, nicht erfindungsgemäß,
- Figur 7: einen für eine Anbringung einer Schutzvorrichtung gemäß Figur 6 bestimmten Handgriff,
- Figur 8: eine an einem Handgriff angebrachte Schutzvorrichtung gemäß Figur 6,
- Figur 9: eine spezielle Ausführungsform der Schutzvorrichtung gemäß Figur 6,
- Figur 10: ein Teil der Schutzvorrichtung gemäß Figur 9,
- Figur 11: spezielle Ausführungsformen eines Teils der Schutzvorrichtung gemäß Figur 6 oder Figur 9 sowie
- Figur 12: eine alternative Ausführungsform eines für eine Anbringung einer Schutzvorrichtung gemäß Figur 6 bestimmten Handgriffs.

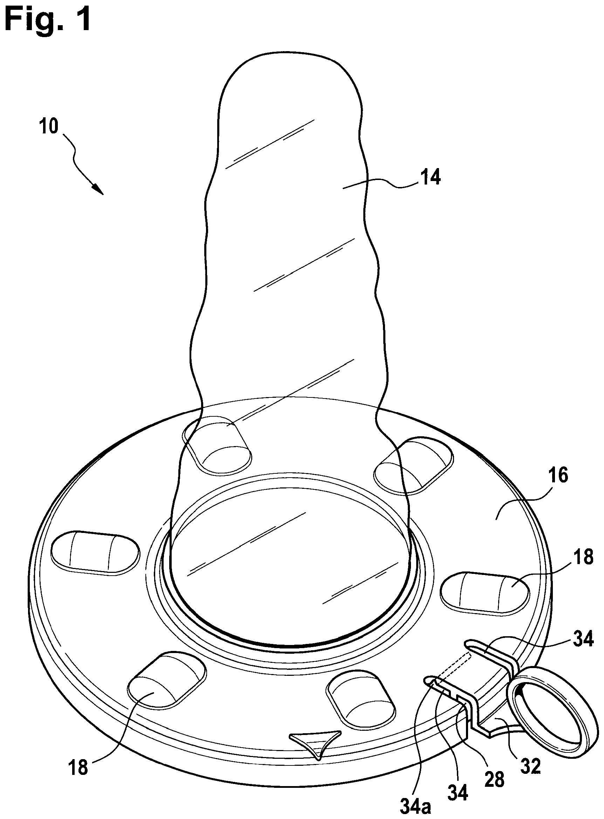

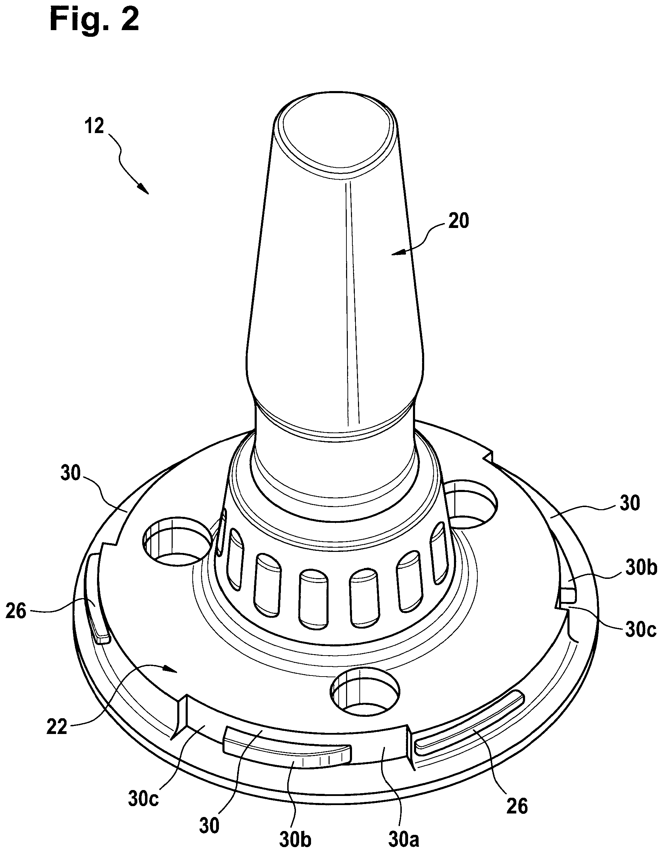

Die Darstellung in Figur 1 zeigt exemplarisch eine Ausführungsform der hier vorgeschlagenen Schutzvorrichtung 10 für einen Handgriff 12 (Fig. 2) eines selbst nicht gezeigten Medizingeräts, zum Beispiel eines Medizingeräts in Form einer OP-Leuchte oder einer Monitoraufnahme. Die Schutzvorrichtung 10 umfasst zwei miteinander verbundene Teile, nämlich ein erstes, flexibles Teil 14, welches über den Handgriff 12 gestülpt wird und im Folgenden kurz als Tüte 14 bezeichnet wird (genauso aber zum Beispiel auch als Beutel bezeichnet werden könnte), sowie ein zweites, festes (starres) Teil 16, mittels dessen die Schutzvorrichtung 10 an dem jeweiligen Handgriff 12 fixiert wird und das im Folgenden als Montageteller 16 bezeichnet wird.

Der Montageteller 16 und die Tüte 14 bestehen vorzugsweise aus einem Kunststoff. Die Tüte 14 ist mit dem Montageteller 16 fest verbunden. Die Verbindung kann zum Beispiel durch Verschweißen, Verkleben oder mittels eines geeigneten Zusatzbauteils durch Klemmen gegeben sein. Der Handgriff 12 weist einen Griffbereich 20 sowie einen Basisbereich 22 auf. Zumindest der Griffbereich 20 des Handgriffs 12 ist bei einer an dem Handgriff 12 angebrachten Schutzvorrichtung 10 komplett mittels der Tüte 14 der Schutzvorrichtung 10 abgedeckt. Die aus einer Sterilverpackung entnommene Schutzvorrichtung 10 wird dabei mit dem Handgriff 12 lösbar verbunden, indem die Tüte 14 über den Griffbereich 20 gestülpt und der Montageteller 16 lösbar am Basisbereich 22 angebracht wird.

Am Montageteller 16 weist die Schutzvorrichtung 10 optionale Drehhilfen 18 auf, in oder an denen die Finger eines Anwenders Halt finden, um den Montageteller 16 der Schutzvorrichtung 10 bei der Anbringung an dem jeweiligen Medizingerät zu drehen. Diese Drehhilfen 18 können gegenüber der angrenzenden Oberfläche des Montagetellers 16 erhaben und/oder versenkt ausgeführt sein.

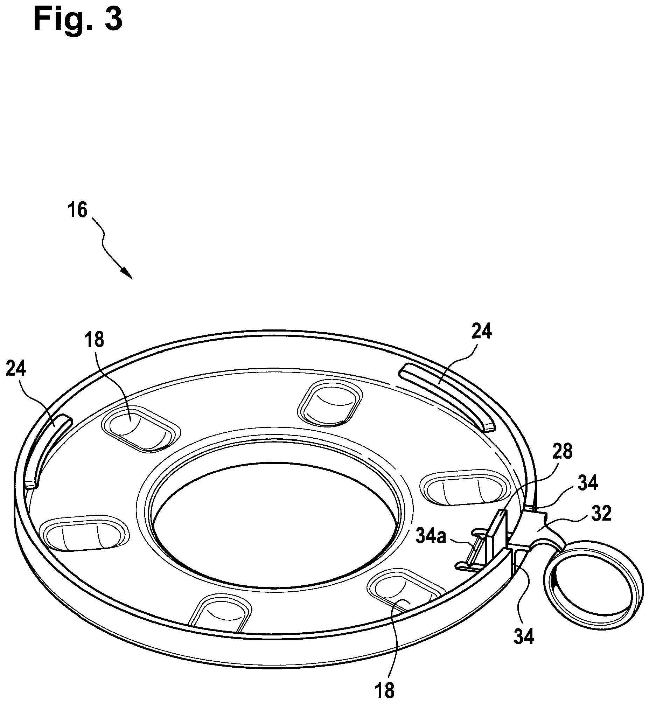

Die Darstellung in Figur 3 zeigt die Unterseite des Montagetellers 16. Mittels eines dabei erkennbaren, vom Montageteller 16 umfassten Profils oder Gewindes 24 ist die Schutzvorrichtung 10 formschlüssig mit dem Handgriff 12 des jeweiligen Medizingeräts verbindbar und wird beim Anbringen an einem Handgriff 12 formschlüssig mit diesem verbunden. Der Handgriff 12 oder das Medizingerät weisen dafür ein zum Profil oder Gewinde 24 der Schutzvorrichtung 10 korrespondierendes Profil oder Gewinde 26 (Fig. 2) auf. Bei dem Gewinde 24 im Montageteller 16 und dem Gewinde 26 am Basisbereich 22 des Handgriffs 12 muss es sich nicht notwendig um ein fortlaufend wendelartig umlaufendes Gewinde 24, 26 handeln. Tatsächlich reichen für eine formschlüssige Verbindung zueinander passende, kurze Schraubenlinienabschnitte aus, wie dies bei der dargestellten Ausführungsform der Fall ist. Alternativ kommt als Profil für eine formschlüssige Verbindung zum Beispiel ein Profil nach Art eines Bajonettverschlusses oder in Form eines Bajonettverschlusses in Betracht. Im Interesse einer besseren Lesbarkeit wird die hier vorgelegte Beschreibung am Beispiel eines Gewindes 24 (Innengewinde), insbesondere eines Innengewindes 24 in Form mehrerer kurzer Schraubenlinienabschnitte, auf der Seite des Montagetellers 16 und eines passenden Gewindes 26 auf der Seite des Handgriffs 12 oder des Medizingeräts fortgesetzt. Andere formschlüssige Verbindungsmöglichkeiten, insbesondere eine Verbindung in Form von oder nach Art eines Bajonettverschlusses, sind dabei stets mitzulesen und ausdrücklich als von der hier vorgelegten Beschreibung mit umfasst anzusehen.

Bei einer optionalen Ausführungsform umfasst der Montageteller 16 der Schutzvorrichtung 10 ein Verriegelungselement 28, das bei der gezeigten Ausführungsform in Form einer Verriegelungsfahne (Fig. 3) realisiert ist. Das Verriegelungselement 28, insbesondere die Verriegelungsfahne, ist dazu bestimmt, die Schutzvorrichtung 10 in einer Endstellung der Drehbewegung auf dem Handgriff 12 zu verriegeln und damit eine ungewollte Demontage zu verhindern. Dazu greift das Verriegelungselement 28, insbesondere rastend, in einen dafür bestimmten Verriegelungsbereich 30 (Fig. 2) im Handgriff 12 ein, insbesondere in einen Verriegelungsbereich 30 in Form einer Ausnehmung im Gewinde 26 des Handgriffs 12.

Optional ist der Verriegelungsbereich 30 so ausgestaltet, dass mittels des Verriegelungsbereichs 30 das Verriegelungselement 28 des Montagetellers 16 beim Drehen des Montagetellers 16 bis in eine Endposition geführt und in dieser verriegelt wird. Dafür ist bei der gezeigten Ausführungsform der Verriegelungsbereich 30 entlang der Außenumfangsfläche des Basisbereichs 22 des Handgriffs 12 um ein Mehrfaches länger als die Breite der als Verriegelungselement 28 fungierenden Verriegelungsfahne. Konkret unterteilt sich der Verriegelungsbereich 30 in einen Fügebereich 30a, einen Anlaufbereich mit einer Anlaufschräge 30b und einen Rastbereich 30c.

Der Fügebereich 30a und der Rastbereich 30c sind entlang der Außenumfangsfläche des Basisbereichs 22 des Handgriffs 12 geringfügig breiter als die Verriegelungsfahne, zumindest so breit, dass der Fügebereich 30a und der Rastbereich 30c die Verriegelungsfahne (Verriegelungselement 28) aufnehmen können.

Beim Anbringen der Schutzvorrichtung 10 und beim Kombinieren von deren Montageteller 16 mit dem Basisbereich 22 des Handgriffs 12 ist oder wird der Montageteller 16 zunächst relativ zum Basisbereich 22 so orientiert, dass die Verriegelungsfahne in den Fügebereich 30a des Verriegelungsbereichs 30 gelangt. Nur dann kann der Montageteller 16 vollständig auf den Basisbereich 22 aufgesetzt werden, so dass das Gewinde 24 des Montagetellers 16 mit dem Gewinde 26 des Basisbereichs 22 zum Eingriff kommt. Beim anschließenden Drehen des Montagetellers 16 relativ zum Basisbereich 22 des Handgriffs 12 wird das Gewinde 24, 26 zunehmend fest und gleichzeitig gelangt die Verriegelungsfahne in den Anlaufbereich und gleitet entlang der Anlaufschräge 30b, wobei das freie Ende der Verriegelungsfahne im Rahmen von deren Materialelastizität radial nach außen ausgelenkt (unter reversibler Verformung nach außen gedrückt) wird. Beim fortgesetzten Weiterdrehen des Montagetellers 16 auf dem Basisbereich 22 gelangt die Verriegelungsfahne schließlich in den Rastbereich 30c. Dabei verlässt die Verriegelungsfahne die Anlaufschräge 30b und federt ohne die bisherige Auslenkung aufgrund der Anlaufschräge 30b in den Rastbereich 30c ein. Der Rastbereich 30c ist in Richtung der bisherigen Drehung durch das Ende des Verriegelungsbereichs 30 und in Gegenrichtung durch das Ende der Anlaufschräge 30b begrenzt. Dieses Ende der Anlaufschräge 30b ist nicht angeschrägt, so dass die in den Rastbereich 30c eingerastete Verriegelungsfahne das Weiterdrehen des Montagetellers 16, aber auch ein Zurückdrehen des Montagetellers 16 verhindert. Der Montageteller 16 ist damit in einer durch den Verriegelungsbereich 30 definierten Endposition am Handgriff 12, nämlich an dessen Basisbereich 22, fixiert, wodurch insgesamt die Schutzvorrichtung 10 am Handgriff 12 fixiert ist.

Optional ist der Verriegelungsbereich 30 mehrfach auf dem Umfang des Basisbereichs 22 vorhanden, insbesondere in einer das Gewinde 24 unterbrechenden Art und Weise. Bei der gezeigten Ausführungsform weist der Basisbereich 22 des Handgriffs 12 mehrere äquidistante Verriegelungsbereiche 30 auf, nämlich drei Verriegelungsbereiche 30, und mit den drei Verriegelungsbereichen 30 wechseln sich entlang des Außenumfangs des Basisbereichs 22 drei Bereiche mit jeweils einem Abschnitt des Gewindes 26 ab.

Bei einer speziellen Variante dieser Ausführungsform dient das Verriegelungselement 28 als fester Anschlag, um bei der durch "Aufschrauben" des Montagetellers 16 auf den Basisbereich 22 erfolgenden Anbringung der Schutzvorrichtung 10 an dem Handgriff 12 eine eindeutige Endstellung des Montagetellers 16 relativ zum Basisbereich 22 zu definieren.

Zum Entriegeln einer an einem Handgriff 12 fixierten Schutzvorrichtung 10 ist an der Schutzvorrichtung 10 eine Entriegelungsvorrichtung 32 vorgesehen, die zur leichteren Handhabung zum Beispiel einen Griffring an deren Ende aufweisen kann. Mittels der Entriegelungsvorrichtung 32 wird das Verriegelungselement 28 außer Eingriff mit dem Verriegelungsbereich 30 gebracht. Ohne das im Verriegelungsbereich 30 fixierte, insbesondere eingerastete Verriegelungselement 28 zu betätigen, kann der Montageteller 16 auch nicht mittels einer Drehbewegung vom Handgriff 12 gelöst und damit die Schutzvorrichtung 10 insgesamt nicht vom Handgriff 12 entfernt werden.

Bei einer besonderen Ausführungsform wird bei der Entriegelung ein Bereich um die Entriegelungsvorrichtung 32 irreversibel deformiert, zum Beispiel eingerissen. Dies wird durch eine geeignete Ausführung des Montagetellers 16 in diesem Bereich unterstützt, zum Beispiel in Form zumindest einer Sollbruchstelle 34a in diesem Bereich, insbesondere einer Sollbruchstelle 34 in Form einer Kerbe oder einer sonstigen Materialschwächung. Bei der gezeigten Ausführungsform schließt die Sollbruchstelle 34a an zumindest einen Schlitz 34 im Montageteller 16 an, nämlich an einen Schlitz 34 oder zwei parallele oder im Wesentlichen parallele Schlitze 34, der bzw. die eine Beweglichkeit der Entriegelungsvorrichtung 32 ermöglicht bzw. ermöglichen. Beim Aufbrechen der Sollbruchstelle 34 ist der Montageteller 16 nach der Entriegelung mittels der Entriegelungsvorrichtung 32 irreversibel deformiert. Der Anwender kann damit die Schutzvorrichtung 10 als "bereits benutzt" erkennen. Somit wird eine Wiederverwendung der als Einmalprodukt vorgesehenen Schutzvorrichtung 10 wirkungsvoll verhindert werden.

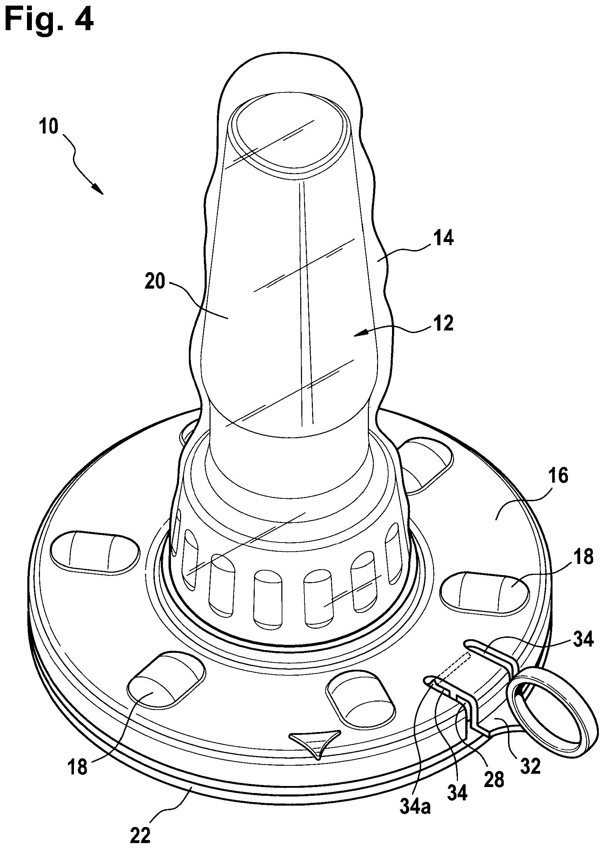

Die Darstellung in Figur 4 zeigt eine an einem Handgriff 12 angebrachte Schutzvorrichtung 10 gemäß Figur 1. Die Tüte 14 der Schutzvorrichtung 10 schließt den Griffbereich 20 des Handgriffs 12 vollständig ein und schließt diesen damit vollständig ab. Der Montageteller 16 der Schutzvorrichtung 10 ist am Basisbereich 22 des Handgriffs 12 angebracht, indem sich dessen Gewinde 24 mit dem Gewinde 26 am Basisbereich 22 in Eingriff befindet. Bei einer Schutzvorrichtung 10 mit einem Verriegelungselement 28 und einer Entriegelungsvorrichtung 32 ist das Verriegelungselement 28 bei einer bestimmungsgemäß am Handgriff 12 angebrachten Schutzvorrichtung 10 in dem oder einem Verriegelungsbereich 30 des Basisbereichs 22 fixiert und mittels der Entriegelungsvorrichtung 32 aus dieser Fixierung lösbar.

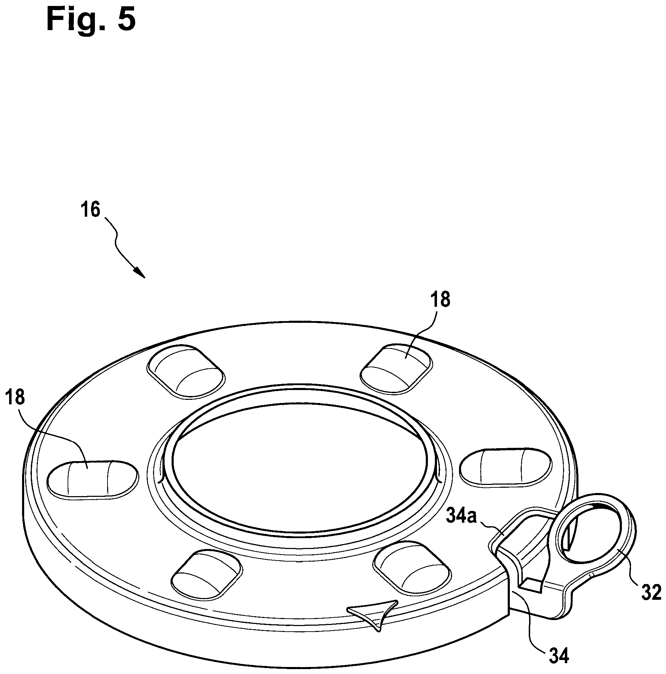

Die Darstellung in Figur 5 zeigt eine spezielle Ausführungsform des Montagetellers 16, wobei die bei einer kompletten Schutzvorrichtung 10 am Montageteller 16 angebrachte Tüte 14 (Figur 1) nicht gezeigt ist. Die Besonderheit dieser Ausführungsform besteht darin, dass die Entriegelungsvorrichtung 32 in Richtung der Drehbewegung des Montagetellers 16 beim Entfernen der Schutzvorrichtung 10 von einem Handgriff 12 ausgerichtet ist. Somit wird nach der Entriegelung mittels der Entriegelungsvorrichtung 32 der Montageteller 16 bereits in die für das Abnehmen der Schutzvorrichtung 10 notwendige Richtung gedreht.

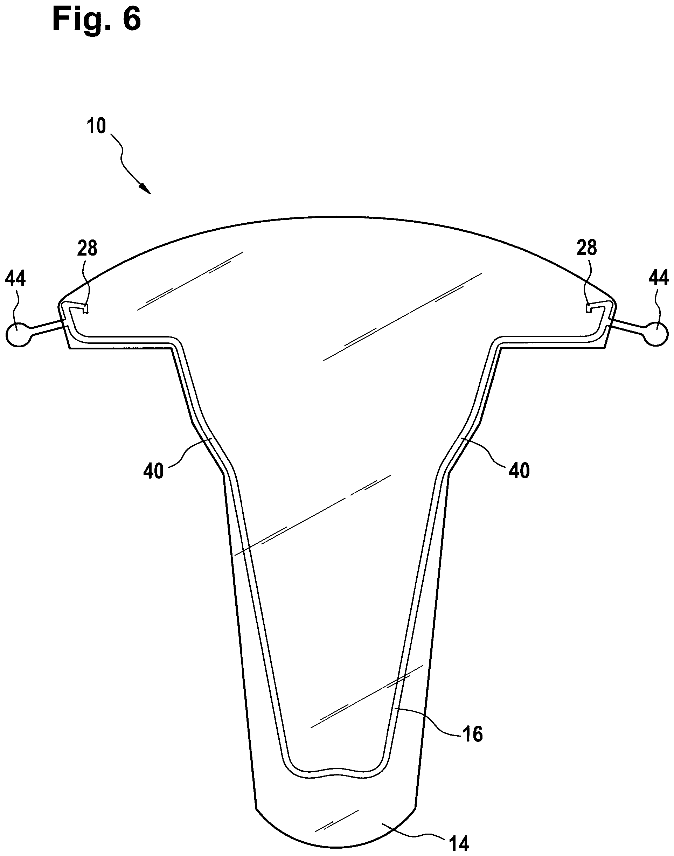

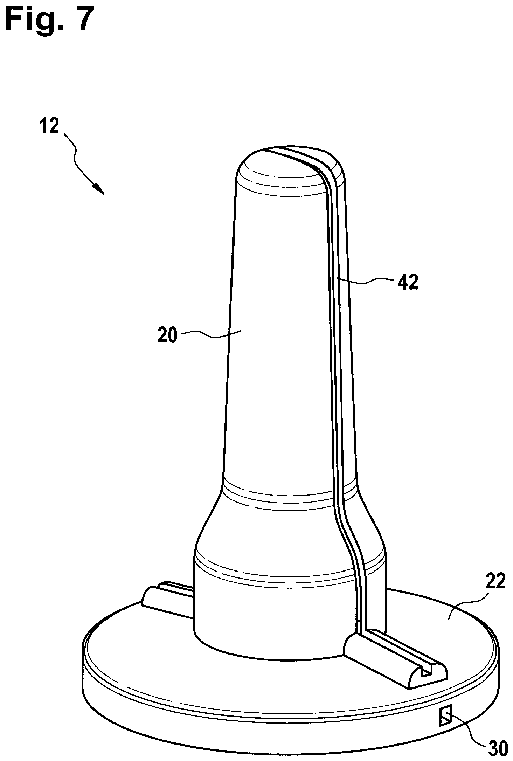

Die Darstellungen in Figur 6 bis Figur 12 zeigen eine weitere Ausführungsform einer Schutzvorrichtung 10 der hier vorgeschlagenen Art für einen Handgriff 12 (Fig. 7) eines auch hier selbst nicht dargestellten Medizingeräts, zum Beispiel eines Medizingeräts in Form einer OP-Leuchte oder einer Monitoraufnahme. Auch diese Schutzvorrichtung 10 weist einen im Folgenden als Tüte 14 bezeichneten flexiblen Teil 14 sowie einen festen Teil 16 auf, der bei dieser Ausführungsform als Klammer 16 ausgeführt ist. Die Klammer 16 ist zum Beispiel aus Metall oder einem Kunststoff gefertigt und jedenfalls federnd elastisch oder zumindest abschnittsweise federnd elastisch ausgeführt. Die Tüte 14 ist zumindest in Teilbereichen 40 fest mit der Klammer 16 verbunden, zum Beispiel verschweißt, verklebt oder verklemmt.

Die Schutzvorrichtung 10 gemäß Figur 6 ist zur Verwendung mit einem Handgriff 12 gemäß Figur 7 vorgesehen. Dieser weist eine Führung 42 für die Schutzvorrichtung 10 auf. Die Führung 42 beginnt im Griffbereich 20 des Handgriffs 12, insbesondere am freien, oberen Ende des Griffbereichs 20, und reicht bis zum Basisbereich 22 des Handgriffs 12. Zum Anbringen der Schutzvorrichtung 10 an einem solchen Handgriff 12 entnimmt der Anwender die Schutzvorrichtung 10 aus einer jeweiligen Sterilverpackung und durch leichtes Zusammendrücken der Klammer 16 öffnet sich die Tüte 14. Damit lässt sich die Schutzvorrichtung 10 über den Handgriff 12 schieben, wobei die Tüte 14 durch den Griffbereich 20 des Handgriffs 12 gegebenenfalls weiter aufgedrückt wird. Bei einer bestimmungsgemäß am Handgriff 12 angebrachten Schutzvorrichtung 10 schließt die Tüte 14 den Griffbereich 20 vollständig ein und deckt diesen damit vollständig ab. Die Klammer 16 wird dabei mittels der Führung 42 des Handgriffs 12 geführt und die Tüte 14 drückt durch die Eigenspannung die Klammer 16 in die Führung 42. Die Führung 42 weist zum Beispiel ein rundes, V-förmiges oder eckiges Profil auf. Alternativ kann eine Führung der Klammer 16 auch mittels einer Nut in der Klammer 16 und einer korrespondierenden Feder als Führung 42 am Handgriff 12 oder einer Feder in der Klammer 16 und einer korrespondierenden Nut als Führung 42 am Handgriff 12 gewährleistet sein.

In der Endposition verriegelt sich die Klammer 16 mit dem Basisbereich 22 des Handgriffs 12 mittels eines von der Klammer 16 umfassten Verriegelungselements 28. Bei der Ausführungsform gemäß Figur 6 fungieren als Verriegelungselement 28 zwei Verriegelungshaken, die in jeweils einen Verriegelungsbereich 30 am Fuß des Handgriffs 12, insbesondere in dessen Basisbereich 22, eingreifen. Zum Abnehmen der Schutzvorrichtung 10 von einem Handgriff 12 nach Ende der Verwendung werden die Verriegelungshaken mittels seitlicher Hilfsgriffe 44 außer Eingriff mit dem Verriegelungsbereich 30 gebracht und die Schutzvorrichtung 10 kann vom Handgriff 12 abgezogen werden.

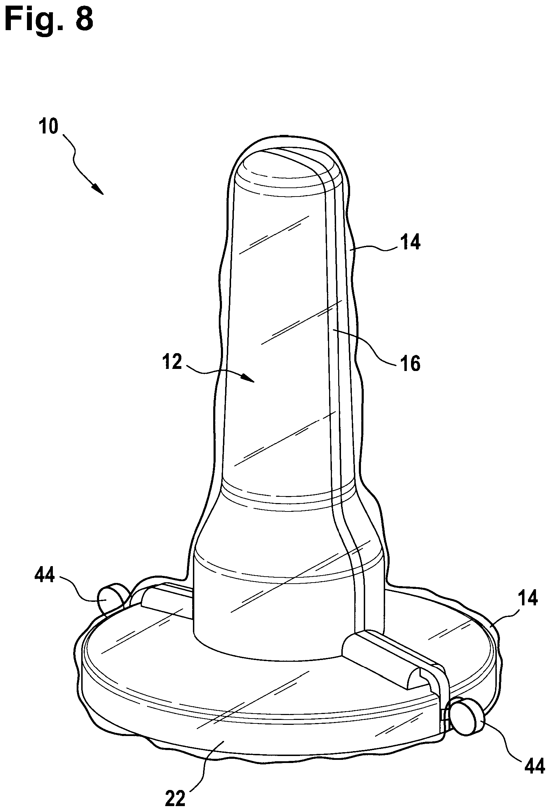

Die Darstellung in Figur 8 zeigt eine an einem Handgriff 12 gemäß Figur 7 angebrachte Schutzvorrichtung 10 gemäß Figur 6. Man erkennt im Innern der Tüte 14 die Klammer 16 in der Führung 42 am Handgriff 12 sowie die Hilfsgriffe 44 zum Lösen der Verrastung der Klammer 16 am Handgriff 12.

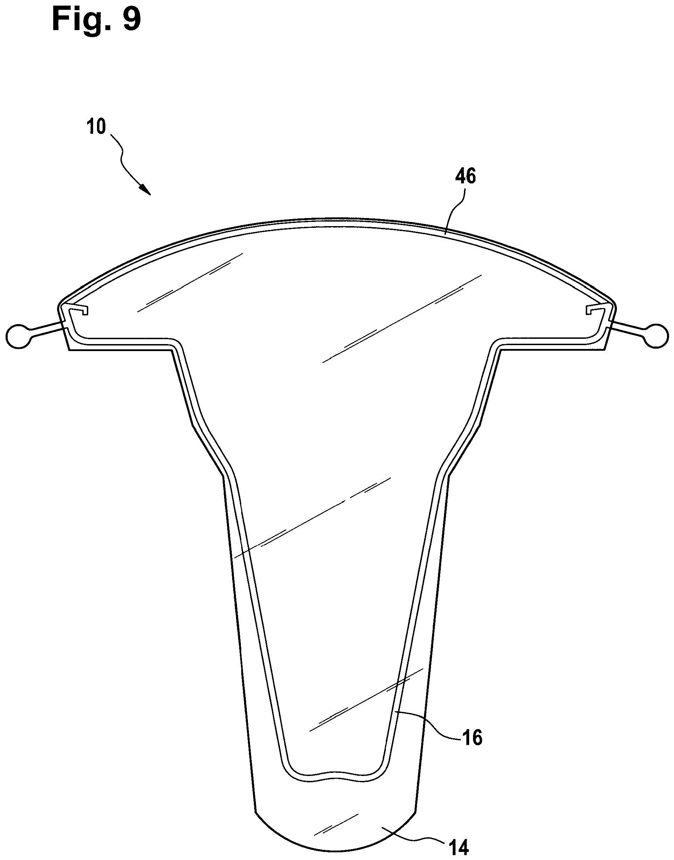

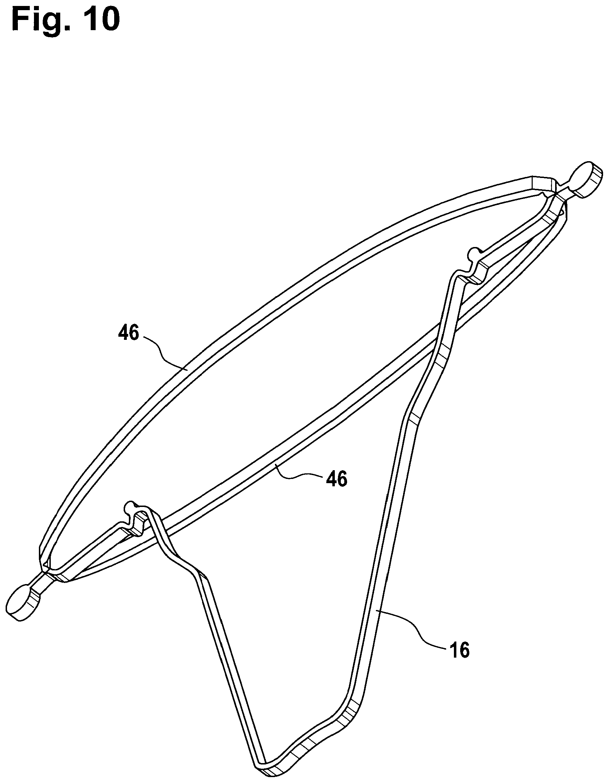

Die Darstellungen in Figur 9 und Figur 10 zeigen eine spezielle Ausführungsform der Schutzvorrichtung 10 gemäß Figur 6. Hier befinden sich an den freien Enden der Klammer 16 zwei Bügel 46, von denen bei der Darstellung der zusammengelegten Schutzvorrichtung 10 in Figur 9 nur einer erkennbar ist. Dadurch wird auch deutlich, dass die Bügel 46 bei einer sich in einer Verpackung (Sterilverpackung) befindlichen Schutzvorrichtung 10 flach liegen und sich erst beim Anbringen der Schutzvorrichtung 10 an einem Handgriff 12 eines Medizingeräts aufstellen.

Die Tüte 14 einer solchen Schutzvorrichtung 10 ist mit ihrem Rand an der offenen Seite zumindest abschnittsweise mit jeweils einem der Bügel 46 verbunden, zum Beispiel durch Verschweißen oder Verkleben. Jeder Bügel 46 ist bereits im entspannten Zustand bogenförmig, wobei die Bogenform eine Vorzugsrichtung beim späteren Verformen beim Anbringen der Schutzvorrichtung 10 an einem Handgriff 12 vorgibt. Beim Anbringen der Schutzvorrichtung 10 an einem Handgriff 12 wird wie bei der Ausführungsform gemäß Figur 6 die Tüte 14 durch Zusammendrücken der Schenkel der Klammer 16 geöffnet. Die beim Zusammendrücken der Schenkel der Klammer 16 auf die Enden der beiden Bügel 46 ausgeübte Kraft bewirkt deren Verformung, so dass sich die Bügel 46 in Richtung auf eine Bogenform mit einem zunehmend kleineren Radius verformen. Im an dem Handgriff 12 angebrachten Zustand bilden die beiden Bügel 46 eine Kreisform oder näherungsweise eine Kreisform oder zumindest eine Form, die der Form der Grundfläche des Basisbereichs 22 des Handgriffs 12 entspricht. Die Bügel 46 fixieren die Ränder der Tüte 14 am Basisbereich 22 des Handgriffs 12, so dass eine möglichst vollständige Abdeckung des Handgriffs 12 gewährleistet ist, zumindest eine vollständige Abdeckung von dessen Griffbereich 20.

Die Beweglichkeit der Bügel 46 umfasst ein Umklappen aus der Ebene der Klammer 16 (Fig. 9) in eine Ebene senkrecht oder zumindest im Wesentlichen senkrecht zu der Ebene der Klammer 16 (Fig. 10) sowie eine elastische Biegsamkeit. Die Beweglichkeit zum Umklappen aus der Verpackungsposition in die Ebene senkrecht oder zumindest im Wesentlichen senkrecht zu der Ebene der Klammer 16 wird mittels geeigneter Scharniere, zum Beispiel Filmscharniere, an den Verbindungsstellen der Bügel 46 mit der Klammer 16 gewährleistet. Die Biegsamkeit der Bügel 46 wird mittels einer geeigneten Materialauswahl gewährleistet. Die Bügel 46 sind zum Beispiel aus einem Kunststoff gefertigt.

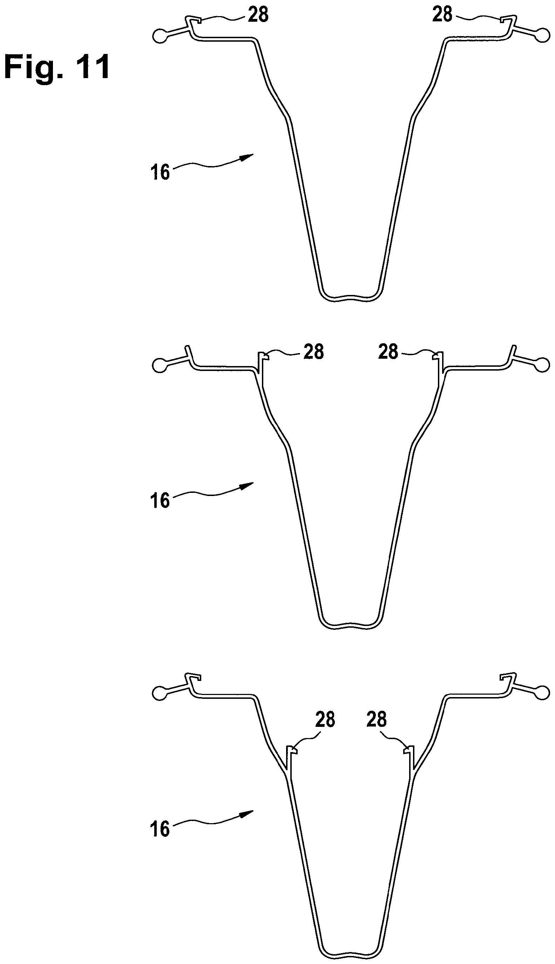

Die Darstellung in Figur 11 zeigt unterschiedliche Positionen für jeweils paarweise angeordnete Verriegelungselemente 28 an einer Klammer 16 der Schutzvorrichtung 10. Der Handgriff 12 weist je nach Klammervariante entsprechende Verriegelungsbereiche 30 auf. Die in Figur 11 gezeigten Verriegelungselemente 28 können einzeln oder paarweise auch mit weiteren Verriegelungselementen 28 kombiniert werden, zum Beispiel derart, dass eine Klammer 16 alle in Figur 11 gezeigten Verriegelungselemente 28 oder einzelne in Figur 11 gezeigte Verriegelungselemente 28 aufweist.

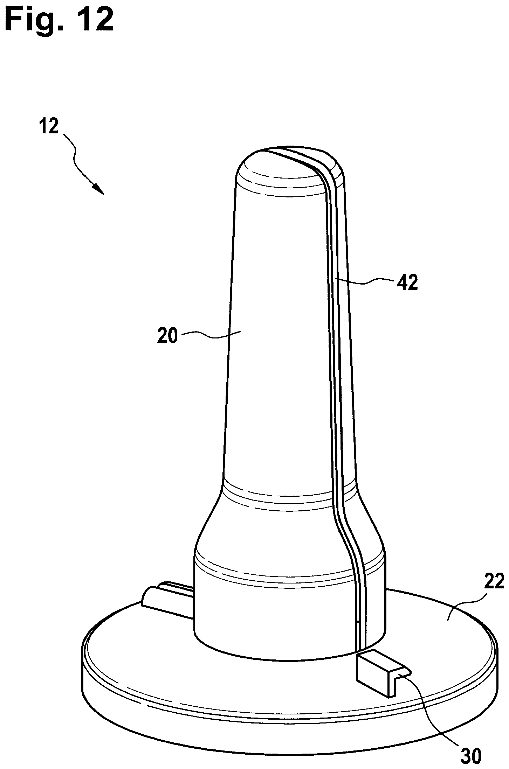

Die Darstellung in Figur 12 zeigt abschließend eine Variante des Handgriffs 12, bei welcher der Verriegelungsbereich 30 in Form eines Hakens am Handgriff 12 ausgeführt ist, zum Beispiel in Form eines Hakens am Basisbereich 22 des Handgriffs 12. Die Schutzvorrichtung 10 wird bei einem solchen Verriegelungsbereich 30 am Handgriff 12 fixiert, indem der betreffende Abschnitt der Klammer 16 in den Haken eingehakt wird.

Einzelne im Vordergrund stehende Aspekte der hier eingereichten Beschreibung lassen sich damit kurz wie folgt zusammenfassen: Angegeben werden eine als Schutzüberzug für ein Teil 12 eines Medizingeräts fungierende Schutzvorrichtung 10 mit einem flexiblen Teil 14 und einem festen Teil 16, wobei das feste Teil 16 wenigstens ein Verriegelungselement 28 aufweist, das mit einem Gegenstück des jeweiligen Teils 12 des Medizingeräts rastend verbindbar ist, sowie ein System, welches eine solche Schutzvorrichtung 10 und einen zum Anbringen einer solchen Schutzvorrichtung 10 bestimmten und eingerichteten Handgriff 12 eines Medizingeräts umfasst.

### BEZUGSZEICHENLISTE

- 10: Schutzvorrichtung
- 12: Handgriff
- 14: flexibles Teil der Schutzvorrichtung / Tüte
- 16: festes Teil der Schutzvorrichtung / Montageteller oder Klammer
- 18: Drehhilfe
- 20: Griffbereich
- 22: Basisbereich
- 24: Gewinde
- 26: Gewinde
- 28: Verriegelungselement
- 30: Verriegelungsbereich
- 30a: Fügebereich
- 30b: Anlaufschräge
- 30c: Rastbereich
- 32: Entriegelungsvorrichtung
- 34: Schlitz
- 34a: Sollbruchstelle
- 36-38: (frei)
- 40: Teilbereich (der Klammer)
- 42: Führung
- 44: Hilfsgriff
- 46: Bügel

## Patentansprüche

1. Schutzvorrichtung (10) für einen Handgriff (12) eines Medizingeräts,
wobei die Schutzvorrichtung (10) ein erstes, flexibles Teil (14) und ein zweites, festes Teil (16) umfasst,
wobei das flexible Teil (14) bei einer am Handgriff (12) angebrachten Schutzvorrichtung (10) zumindest einen Griffbereich (20) des Handgriffs (12) vollständig abdeckt und
wobei das feste Teil (16) lösbar mit einem Basisbereich (22) des Handgriffs (12) in Eingriff ist,
**dadurch gekennzeichnet, dass**
das feste Teil (16) zumindest ein Verriegelungselement (28) zur lösbaren Fixierung am Basisbereich (22) umfasst,
ein Profil in Form oder nach Art eines Bajonettverschlusses oder ein Gewinde (24) im festen Teil (16) vorgesehen ist,
welches zum Zusammenwirken mit einem Profil in Form oder nach Art eines Bajonettverschlusses oder einem Gewinde (26) im Basisbereich (22) bestimmt ist,
wobei das feste Teil (12) der Schutzvorrichtung (10) relativ zum Basisbereich (22) des Handgriffs (12) drehbar ist und
wobei das Verriegelungselement (28) die Form einer Verriegelungsfahne aufweist, die dazu ausgestaltet ist, in einen Verriegelungsbereich (30) im Handgriff (12) einzugreifen.

2. Schutzvorrichtung (10) nach Anspruch 1, **dadurch gekennzeichnet, dass** das Verriegelungselement (28) die Schutzvorrichtung (10) in einer Endstellung der Drehbewegung auf dem Handgriff (12) zu verriegeln vermag.

3. Schutzvorrichtung (10) nach Anspruch 1 oder 2, mit Drehhilfen (18) am festen Teil (16).

4. Schutzvorrichtung (10) nach Anspruch 1, 2 oder 3, mit einer zum Lösen des Verriegelungselements (28) aus einer Fixierung im Basisbereich (22) des Handgriffs (12) bestimmten Entriegelungsvorrichtung (32) am festen Teil (16).

5. Schutzvorrichtung (10) nach Anspruch 4, mit zumindest einer der Entriegelungsvorrichtung (32) am festen Teil (16) räumlich zugeordneten Sollbruchstelle (34a).

6. Schutzvorrichtung (10) nach einem der Ansprüche 1 bis 5, mit einem starren, tellerförmigen festen Teil (16).

7. Schutzvorrichtung (10) nach Anspruch 1, mit einer zumindest in Teilbereichen (40) mit dem flexiblen Teil (14) verbundenen Klammer (16) als festem Teil (16).

8. Schutzvorrichtung (10) nach Anspruch 7, mit zumindest einem als Verriegelungselement (28) fungierenden Verriegelungshaken an der Klammer (16).

9. Schutzvorrichtung (10) nach Anspruch 7 oder 8, mit zwei an der Klammer (16) angelenkten und mit dem flexiblen Teil (14) in dessen Randbereich zumindest abschnittsweise verbundenen Bügeln (46).

10. Handgriff für ein Medizingerät mit einer Schutzvorrichtung nach einem der Ansprüche 1 bis 9.

11. System mit einer Schutzvorrichtung (10) nach einem der Ansprüche 1 bis 9 und einem Handgriff (12) eines Medizingeräts,
wobei der Handgriff (12) einen Griffbereich (20) und einen Basisbereich (22) aufweist und
wobei der Basisbereich (22) zur lösbaren Fixierung des festen Teils (16) der Schutzvorrichtung (10) am Handgriff (12) eingerichtet ist, indem der Basisbereich (22) zumindest einen Verriegelungsbereich (30) zur Aufnahme des Verriegelungselements (28) der Schutzvorrichtung (10) aufweist.

12. System nach Anspruch 11, wobei der Verriegelungsbereich (30) einen Fügebereich (30a), einen Rastbereich (30c) und dazwischen einen Anlaufbereich mit einer Anlaufschräge (30b) aufweist,
wobei beim Verbinden der Schutzvorrichtung (10) mit dem Handgriff (12) zunächst der Fügebereich (30a) das Verriegelungselement (28) aufnimmt,
wobei beim Verbinden der Schutzvorrichtung (10) mit dem Handgriff (12) und beim Drehen des festen Teils (16) relativ zum Basisbereich (22) des Handgriffs (12) die Anlaufschräge (30b) das Verriegelungselement (28) auslenkt und
wobei beim Weiterdrehen des festen Teils (16) das Verriegelungselement (28) von der Anlaufschräge (30b) in den Rastbereich (30c) gelangt und dort zur rastenden Fixierung des festen Teils (16) am Basisbereich (22) des Handgriffs (12) wirksam ist.

## Claims

1. Protective device (10) for a handle (12) of a medical device,
wherein the protective device (10) comprises a first, flexible part (14) and a second, rigid part (16),
wherein the flexible part (14) completely covers at least a grip region (20) of the handle (12) when the protective device (10) is mounted on the handle (12), and
wherein the rigid part (16) engages releasably with a base region (22) of the handle (12),
**characterized in that**
the rigid part (16) comprises at least one locking element (28) for releasable fixing to the base region (22),
a profile in the form or in the manner of a bayonet catch or a thread (24) is provided in the rigid part (16),
which profile is provided for interaction with a profile in the form or in the manner of a bayonet catch or a thread (26) in the base region (22),
wherein the rigid part (12) of the protective device (10) is rotatable relative to the base region (22) of the handle (12), and
wherein the locking element (28) has the form of a locking tab which is designed to engage in a locking region (30) in the handle (12).

2. Protective device (10) according to Claim 1, **characterized in that** the locking element (28) is able to lock the protective device (10) on the handle (12) in an end position of the rotational movement.

3. Protective device (10) according to Claim 1 or 2, with rotation aids (18) on the rigid part (16).

4. Protective device (10) according to Claim 1, 2 or 3, with an unlocking device (32) on the rigid part (16), which unlocking device (32) is provided for releasing the locking element (28) from being fixed in the base region (22) of the handle (12).

5. Protective device (10) according to Claim 4, with at least one predetermined breaking point (34a) assigned spatially to the unlocking device (32) on the rigid part (16).

6. Protective device (10) according to one of Claims 1 to 5, with an inflexible, disc-shaped rigid part (16) .

7. Protective device (10) according to Claim 1, with a clip (16), as rigid part (16), connected at least in sub-regions (40) to the flexible part (14).

8. Protective device (10) according to Claim 7, with at least one locking hook, functioning as locking element (28), on the clip (16).

9. Protective device (10) according to Claim 7 or 8, with two bows (46) articulated on the clip (16) and connected at least in some sections to the edge region of the flexible part (14).

10. Handle for a medical device, with a protective device according to one of Claims 1 to 9.

11. System comprising a protective device (10) according to one of Claims 1 to 9 and comprising a handle (12) of a medical device,
wherein the handle (12) has a grip region (20) and a base region (22), and
wherein the base region (22) is configured for releasably fastening the rigid part (16) of the protective device (10) to the handle (12), the base region (22) having at least one locking region (30) for receiving the locking element (28) of the protective device (10).

12. System according to Claim 11, wherein the locking region (30) has a joining region (30a), a latching region (30c) and, between these, a run-on region with a run-on bevel (30b),
wherein the joining region (30a) first receives the locking element (28) during the connection of the protective device (10) to the handle (12),
wherein the run-on bevel (30b) deflects the locking element (28) during the connection of the protective device (10) to the handle (12) and during the rotation of the rigid part (16) relative to the base region (22) of the handle (12), and
wherein, upon further rotation of the rigid part (16), the locking element (28) passes from the run-on bevel (30b) into the latching region (30c) and acts there to effect the latching of the rigid part (16) to the base region (22) of the handle (12).

## Revendications

1. Dispositif de protection (10) pour une poignée (12) d'un appareil médical, le dispositif de protection (10) comprenant une première partie souple (14) et une seconde partie fixe (16),
la partie souple (14) recouvrant complètement au moins une zone de poignée (20) de la poignée (12) lorsque le dispositif de protection (10) est monté sur la poignée (12), et
la partie fixe (16) étant en prise de manière amovible avec une zone de base (22) de la poignée (12),
**caractérisé en ce que**
la partie fixe (16) comprend au moins un élément de verrouillage (28) pour une fixation amovible au niveau de la zone de base (22),
un profil sous la forme ou à la manière d'une fermeture à baïonnette ou un filetage (24) est prévu dans la partie fixe (16),
qui est destiné à coopérer avec un profil sous la forme ou à la manière d'une fermeture à baïonnette ou d'un filetage (26) dans la zone de base (22),
la partie fixe (12) du dispositif de protection (10) pouvant tourner par rapport à la zone de base (22) de la poignée (12), et
l'élément de verrouillage (28) présentant la forme d'une languette de verrouillage, qui est conçue pour venir en prise dans une zone de verrouillage (30) dans la poignée (12).

2. Dispositif de protection (10) selon la revendication 1, **caractérisé en ce que** l'élément de verrouillage (28) est capable de verrouiller le dispositif de protection (10) dans une position finale du mouvement de rotation sur la poignée (12).

3. Dispositif de protection (10) selon la revendication 1 ou 2, comprenant des moyens de rotation (18) sur la partie fixe (16).

4. Dispositif de protection (10) selon la revendication 1, 2 ou 3, comprenant au niveau de la partie fixe (16) un dispositif de déverrouillage (32) destiné à libérer l'élément de verrouillage (28) d'une fixation dans la zone de base (22) de la poignée (12)

5. Dispositif de protection (10) selon la revendication 4, avec au moins un point de rupture de consigne (34a) associé spatialement au dispositif de déverrouillage (32) au niveau de la partie fixe (16).

6. Dispositif de protection (10) selon l'une quelconque des revendications 1 à 5, comprenant une partie fixe rigide en forme de plaque (16).

7. Dispositif de protection (10) selon la revendication 1, comprenant en tant que partie fixe (16) une attache (16) reliée à la partie souple (14) au moins dans des zones partielles (40).

8. Dispositif de protection (10) selon la revendication 7, comprenant au moins un crocher de verrouillage agissant comme un élément de verrouillage (28) au niveau de l'attache (16).

9. Dispositif de protection (10) selon la revendication 7 ou 8, comprenant deux étriers (46) articulés au niveau de l'attache (16) et reliés au moins partiellement à la partie souple (14) dans sa zone de bord.

10. Poignée pour un appareil médical avec un dispositif de protection selon l'une quelconque des revendications 1 à 9.

11. Système comprenant un dispositif de protection (10) selon l'une quelconque des revendications 1 à 9 et une poignée (12) d'un appareil médical,
la poignée (12) présentant une zone de poignée (20) et une zone de base (22) et
la zone de base (22) étant prévue pour une fixation amovible de la partie fixe (16) du dispositif de protection (10) au niveau de la poignée (12), la zone de base (22) présentant au moins une zone de verrouillage (30) pour recevoir l'élément de verrouillage (28) du dispositif de protection (10).

12. Système selon la revendication 11, dans lequel la zone de verrouillage (30) comprend une zone d'assemblage (30a), une zone d'encliquetage (30c) et, entre elles, une zone d'amorce avec une pente d'amorce (30b),
lorsque le dispositif de protection (10) est connecté à la poignée (12), la zone d'assemblage (30a) recevant d'abord l'élément de verrouillage (28),
lorsque le dispositif de protection (10) est connecté à la poignée (12) et lorsque la partie fixe (16) est tournée par rapport à la zone de base (22) de la poignée (12), la rampe d'amorce (30b) déviant l'élément de verrouillage (28), et
lorsque la partie fixe (16) est tournée davantage, l'élément de verrouillage (28) se déplace de la pente d'amorce (30b) jusque dans la zone d'encliquetage (30c) et ensuite est actif pour une fixation par encliquetage de la partie fixe (16) au niveau de la zone de base (22) de la poignée (12).
